# EUROPEAN PATENT APPLICATION

(11) **EP 3 940 383 A1**
(43) Date of publication of application: **19.01.2022**
(21) Application number: 20305821.9
(22) Date of filing: 16.07.2020
(51) Int. Cl.: G01N 33/68

(54) **METHODS FOR PREDICTING THE OUTCOME OF PATIENTS HAVING ACUTE ISCHEMIC STROKE**

(71) Applicant: INSERM (Institut National de la Santé et de la Recherche Médicale), 75013 Paris (FR); Université de Paris, 75006 Paris (FR); Université Paris XIII Paris-Nord, 93430 Villetaneuse (FR); Fondation Ophthalmologique Adolphe De Rothschild, 75019 Paris (FR)
(72) Inventor: The designation of the inventor has not yet been filed
(74) Representative: Inserm Transfert

(57) **Abstract**

Accumulating evidence point to a major role of neutrophils in the pathophysiology of acute ischemic stroke (AIS). Whether and how neutrophil activation evolves following endovascular therapy (EVT) for AIS remains unknown. Here, the inventors investigated correlations between dynamic changes in plasma levels of neutrophil activation markers and AIS outcome in EVT-treated patients. Plasma levels of matrix metalloproteinase-9 (MMP-9), neutrophil elastase (NE), MPO, and citrullinated histone H3 (H3Cit) were measured at admission and at 1h and 24h post-EVT. The results show that plasma levels of MMP-9, MPO and H3Cit continue evolving after successful recanalization by EVT, with an early and transient increase in MPO and H3Cit plasma levels at 1 h post-EVT, and a gradual decrease in MMP-9 plasma level. Higher admission levels of H3Cit were also associated with poor functional outcome. In conclusion, the results show that admission H3Cit level are associated with outcome in AIS patients, and support the notion that neutrophil effectors are informative biomarkers of AIS severity and prognosis.

## Description

### FIELD OF THE INVENTION:

The present invention is in the field of medicine and in particular cardiovascular diseases.

### BACKGROUND OF THE INVENTION:

Endovascular therapy (EVT) in the setting of acute ischemic stroke (AIS) consecutive to large vessel occlusion (LVO) achieves successful recanalization at rates as high as 90%. Nevertheless, despite this tremendous recanalization efficacy, more than 50% of EVT-treated patients still evolve toward a poor functional outcome¹. High admission neutrophil count²⁻⁵ and neutrophil to lymphocyte ratio (NLR)^{3,6-8}, as well as admission hyperglycemia⁹, which primes neutrophil responsiveness to ischemia¹⁰, are critical factors associated with unfavorable functional outcome in AIS patients treated by IV tPA and/or EVT. It is becoming increasingly evident that neutrophils are not only markers of stroke severity, but also active players in stroke pathophysiology. Circulating levels of neutrophil effectors like free myeloperoxidase (MPO) or neutrophil extracellular traps (NETs), which are released upon neutrophil activation, have been shown to be positively correlated with stroke severity at onset and independently associated with post-stroke all-cause mortality^{3,11,12}. Moreover, recent studies have identified NETs as constitutive components of AIS thrombi causing large LVO¹³⁻¹⁵. There are indications that NETs in AIS thrombi contribute both to the thrombus extracellular scaffold and to its resistance to tPA-mediated thrombolysis¹³⁻¹⁵. Degradation of extracellular DNA by DNase I treatment was indeed shown to improve *ex vivo* tPA-mediated thrombolysis of EVT-retrieved AIS thrombi^{13,14}. Studies in animal models of ischemic stroke have further revealed that LVO rapidly triggers a detrimental neutrophil response characterized by the formation of platelet/neutrophil aggregates occluding microvessels downstream of the occlusion site¹⁶⁻¹⁸. Taken as a whole, all these data show that neutrophil activation is an early event in AIS and indicate that neutrophils and their effectors represent useful prognostic factors but also potential therapeutic targets in AIS.

### SUMMARY OF THE INVENTION:

The present invention is defined by the claims. In particular, the present invention relates to methods for predicting the outcome of patients having acute ischemic stroke.

### DETAILED DESCRIPTION OF THE INVENTION:

Accumulating evidence point to a major role of neutrophils in the pathophysiology of acute ischemic stroke (AIS). Circulating levels of neutrophil effectors like myeloperoxidase (MPO) or neutrophil extracellular traps (NETs) have been found to be positively correlated and independently associated with stroke severity at onset and post-stroke mortality. Moreover, recent studies have shown that NETs contribute to the extracellular scaffold of AIS thrombi and favor thrombolysis resistance. Whether and how neutrophil activation evolves following endovascular therapy (EVT) for AIS remains unknown. Here, the inventors investigated correlations between dynamic changes in plasma levels of neutrophil activation markers and AIS outcome in EVT-treated patients. Plasma levels of matrix metalloproteinase-9 (MMP-9), neutrophil elastase (NE), MPO, and citrullinated histone H3 (H3Cit) were measured at admission and at 1h and 24h post-EVT. The results show that plasma levels of MMP-9, MPO and H3Cit continue evolving after successful recanalization by EVT, with an early and transient increase in MPO and H3Cit plasma levels at 1 h post-EVT, and a gradual decrease in MMP-9 plasma level. NE level remained stable over time. Higher variations in MPO level between admission and 24 h post-EVT were associated with a higher risk of poor 90-day functional outcome, independently of initial stroke severity and neutrophil count. Higher admission levels of H3Cit were also associated with poor functional outcome. In conclusion, the results show that admission H3Cit level and dynamic changes in MPO level are associated with outcome in EVT-treated AIS patients, and support the notion that neutrophil effectors are informative biomarkers of AIS severity and prognosis.

Accordingly the first object of the present invention relates to a method of predicting the outcome of a patient suffering from an acute ischemic stroke (AIS) comprising determining the level of citrullinated histones H3 in a sample obtained from the patient wherein said level indicates the outcome.

As used herein, the term **"stroke"** has its general meaning in the art and refers to an episode of neurological dysfunction caused by focal cerebral, spinal, or retinal infarction (Easton et al., Stroke 2009, 40, 2276-2293). In particular, the term encompasses acute ischemic stroke (AIS), transient ischemic attack (TIA) and hemorrhagic stroke. Acute ischemic stroke can result from a variety of causes such as atherosclerosis of the cerebral circulation, occlusion of cerebral small vessels, and cardiac embolism. Cardiac embolism results from one of three mechanisms: blood stasis and thrombus formation in an enlarged (or affected by another structure alteration) left cardiac chamber (e.g., left ventricular aneurysm); release of material from an abnormal valvular surface (e.g., calcific degeneration); and abnormal passage from the venous to the arterial circulation (paradoxical embolism).

In some embodiments, the method of the present invention is particularly suitable for predicting the outcome of a patient suffering from an acute ischemic stroke (AIS) after endovascular therapy.

As used herein, the term **"endovascular therapy"** or **"EVT"** has its general meaning in the art and refers to the non-surgical treatment for the treatment of AIS and that typically includes any one or more of the following: thrombolysis, clot and stent retrieval and thrombectomy. The EVT typically involves uses of microcatheters (thin tubes visible under X-rays) which are inserted into the blood clot from the groin or the arm. When, the blood clot is removed from the blood vessel - this procedure is called a thrombectomy. If the blood clot cannot be removed, it is liquefied using drugs delivered through the catheter, in a procedure known as thrombolysis. Thrombolysis involves endovascular administration of t-PA. As used herein, the term **"t-PA"** has its general meaning in the art and refers to tissue-type plasminogen activator. The term includes native t-PA and recombinant t-PA, as well as modified forms of t-PA that retain the enzymatic or fibrinolytic activities of native t-PA.

As used herein, the term **"outcome"** includes the survival of the patient after a defined time, e.g. after 5 days, 4 weeks, 3 months, 1 year or re-stroke or to a functional outcome.

The method of the present invention is particularly suitable for predicting the functional outcome of the patient.

As used herein, the term **"functional outcome"** in the context of the present invention relates to the degree of severity of the disease, i.e. the state of health the patient after a defined time, e.g. after 5 days, 4 weeks, 3 months, or 1 year, preferably with regard to the stroke symptoms. In particular, the functional outcome summarizes the neurological impairments, disabilities, and handicaps that occur after AIS. The functional outcome for stroke encompasses a broad range of disabilities and impairments as well as the relationship of disability and impairment to independent function. Typically the method of the present invention is particularly suitable for predicting the functional outcome at 1 month, 3 months or 6 months AIS. Generally, a functional outcome of functional dependence is a poor outcome in which the subject suffers from impairment, disability, handicap or compromised quality of life. The functional outcome is assessed according to the Barthel Index, NIHSS and preferably modified Ranking Scale as depicted in Table A. In particular, a poor clinical outcome is defined as a modified Rankin Scale score of 3 or more.

**Table A: modified Ranking Scale**

| Score | Clinical setting |
|---|---|
| 0 | No symptoms at all |
| 1 | No significant disability despite symptoms; able to carry out all usual duties and activities |
| 2 | Slight disability; unable to carry out all previous activities, but able to look after own affairs without assistance |
| 3 | Moderate disability; requiring some help, but able to walk without assistance |
| 4 | Moderately severe disability; unable to walk and attend to bodily needs without assistance |
| 5 | Severe disability; bedridden, incontinent and requiring constant nursing care and attention |
| 6 | Death |

As used herein, the term **"sample"** refers to any sample obtained from the subject for the purpose of performing the method of the present invention.

According to the present invention the sample is obtained at admission of the patient.

In some embodiments, the sample is a blood sample.

As used herein, the term **"blood sample"** refers to a whole blood sample, serum sample and plasma sample. A blood sample may be obtained by methods known in the art including venipuncture or a finger stick. Serum and plasma samples may be obtained by centrifugation methods known in the art. The sample may be diluted with a suitable buffer before conducting the assay.

As used herein, the term **"Histone H3"** has its general meaning in the art and refers to one of the five main families of histone proteins and represents the most extensively modified of the histone proteins by post-translational modifications. Specific subfamilies of histone H3 include H3A1, H3A2, and H3A3 and include the exemplary H3 histones HIST1H3A, HIST1H3B, HIST1H3C, HIST1H3D, HIST1H3E, HIST1H3F, HIST1H3G, HIST1H3H, HIST1H3I, HIST1H3J, HIST2H3C, and HIST3H3.

As used herein, the term **"citrullinated histone H3"** or **"H3Cit"** refers to a Histone H3 that is citrullinated. Citrullinated histone H3 has been identified as a component of neutrophil extracellular traps (NETs) that are produced by degranulating neutrophils.

As used herein, the term **"citrullination"** refers to modification of arginine to citrulline, which may be a post-translational modification by the enzyme PAD. In particular, citrullination of histones H3 by PAD4 has been shown to target multiple arginine sites in histones H3 (Arg-2, Arg-8, Arg-17, and Arg-26, e.g., Arg-8 and Arg-17; see, e.g., Bauer et al., (2002) EMBO Rep, 3:39-44, and Wang et al. Science 306:279-283, (2004)). PAD4 is an enzyme previously known to convert protein arginine (Arg) to citrulline (Cit), a nonconventional amino acid in proteins (Vossenaar et al., Bioessays. 2003; 25: 1106-1118).

The level of H3Cit in the sample can be determined using methods known in the art, e.g., using quantitative immunoassay methods such as enzyme linked immunosorbent assays (ELISAs), immunoprecipitations, immunofluorescence, enzyme immunoassay (EIA), radioimmunoassay (RIA), and Western blot analysis. In some embodiments, the methods include contacting an agent that selectively binds to the H3Cit protein (such as an antibody or antigen-binding portion thereof) with a sample, to evaluate the level of protein in the sample. In some embodiments, the antibody bears a detectable label. Antibodies can be polyclonal, or more preferably, monoclonal. An intact antibody, or an antigen-binding fragment thereof (e.g., Fab or F(ab')2) can be used.

As used herein, the term **"labeled"** with regard to an antibody encompasses direct labeling of the antibody by coupling (i.e., physically linking) a detectable substance to the antibody, as well as indirect labeling of the antibody by reactivity with a detectable substance. Examples of detectable substances are known in the art and include chemiluminescent, fluorescent, radioactive, or colorimetric labels. For example, detectable substances can include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

In some embodiments, high throughput methods, e.g., protein or gene chips as are known in the art (see, e.g., Ch. 12, "Genomics," in Griffiths et al., Eds. Modern genetic Analysis, 1999, W. H. Freeman and Company; Ekins and Chu, Trends in Biotechnology, 1999; 17:217-218; MacBeath and Schreiber, Science 2000, 289(5485):1760-1763; Simpson, Proteins and Proteomics: A Laboratory Manual, Cold Spring Harbor Laboratory Press; 2002; Hardiman, Microarrays Methods and Applications: Nuts & Bolts, DNA Press, 2003), can be used to detect the presence and/or level of H3Cit.

In some embodiments, microfluidic (e.g., "lab-on-a-chip," "micro-a-fluidic chips") devices can be used in the present methods for detection and quantification of H3Cit protein in a sample. Such devices have been successfully used for microfluidic flow cytometry, continuous size-based separation, and chromatographic separation. In particular, such devices can be used for the isolation of specific biological particles such as specific proteins (e.g., H3Cit) from complex mixtures such as serum (e.g., whole blood, serum, or plasma. A variety of approaches may be used to separate H3Cit proteins from a heterogeneous sample. For example, some techniques can use functionalized materials to capture H3Cit using functionalized surfaces that bind to the target cell population. The functionalized materials can include surface-bound capture moieties such as antibodies or other specific binding molecules, such as aptamers, as are known in the art. Accordingly, such microfluidic chip technology may be used in diagnostic and prognostic devices for use in the methods described herein. For examples, see, e.g., Lion et al., Electrophoresis 24 21 3533-3562 (2003); Fortier et al., Anal. Chem., 77(6): 1631-1640 (2005); U.S. Patent Publication No. 2009/0082552; and U.S. Pat. No. 7,611,834. Also included in the present application are microfluidics devices comprising H3Cit binding moieties, e.g., anti-H3Cit antibodies or antigen-binding fragments thereof.

Typically, high levels of H3Cit indicate a poor outcome (e.g. poor functional outcome) and conversely low levels of H3Cit indicate a good outcome (e.g. good functional outcome).

As used herein, the term **"high"** refers to a measure that is greater than normal, greater than a standard such as a predetermined reference value or a subgroup measure or that is relatively greater than another subgroup measure. For example, high H3Cit refers to a measure of H3Cit that is greater than a normal H3Cit measure. A normal H3Cit measure may be determined according to any method available to one skilled in the art. High H3Cit may also refer to a measure that is equal to or greater than a predetermined reference value, such as a predetermined cutoff. High H3Cit may also refer to a measure of H3Cit wherein a high H3Cit subgroup has relatively greater levels of H3Cit than another subgroup. For example, without limitation, according to the present specification, two distinct patient subgroups can be created by dividing samples around a mathematically determined point, such as, without limitation, a median, thus creating a subgroup whose measure is high (i.e., higher than the median) and another subgroup whose measure is low. In some cases, a "high" level may comprise a range of level that is very high and a range of level that is "moderately high" where moderately high is a level that is greater than normal, but less than "very high".

As used herein, the term **"low"** refers to a measure that is less than normal, less than a standard such as a predetermined reference value or a subgroup measure that is relatively less than another subgroup measure. For example, low H3Cit means a measure of H3Cit that is less than a normal H3Cit measure in a particular set of samples of patients. A normal H3Cit measure may be determined according to any method available to one skilled in the art. Low H3Cit may also mean a measure that is less than a predetermined reference value, such as a predetermined cutoff. Low H3Cit may also mean a measure wherein a low H3Cit subgroup is relatively lower than another subgroup. For example, without limitation, according to the present specification, two distinct patient subgroups can be created by dividing samples around a mathematically determined point, such as, without limitation, a median, thus creating a group whose measure is low (i.e., less than the median) with respect to another group whose measure is high (i.e., greater than the median).

In some embodiments, the method of the present invention comprises the steps of i) quantifying the level of H3Cit in the sample obtained from the patient ii) comparing the level quantified at step i) with a predetermined reference value and iii) concluding that the patient will have a poor outcome when the level quantified at step i) is higher than the predetermined reference value or inversely concluding that the patient will have a good outcome when the content quantified at step i) is lower than the predetermined reference value.

In some embodiments, the predetermined reference value is a threshold value or a cut-off value that can be determined experimentally, empirically, or theoretically. A threshold value can also be arbitrarily selected based upon the existing experimental and/or clinical conditions, as would be recognized by a person of ordinary skilled in the art. For example, retrospective measurement in properly banked historical subject samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after quantifying the H3Cit level in the sample, one can use algorithmic analysis for the statistic treatment of the H3Cit level determined in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc.

The method of the present invention is particularly suitable for identifying patients that may need extra attention and support after EVT.

The method of the present invention is also particularly suitable to determine whether the patient is eligible to a particular therapy. In particular, the method of the present invention is suitable for determining whether the patient is eligible with a therapy with DNAse. In particular, patient having a poor outcome can then be administered with a therapeutically effective amount of DNAse.

As used herein, the term **"DNase"** has its general meaning in the art and refers to and includes all enzymes having a phosphodiesterase activity and the ability to hydrolyse DNA. Any suitable DNase may be used in the present invention. The DNase will most preferably be a DNase I (EC 3.1.21.1). It may, however, in some embodiments be a DNase II (EC 3.1.21.1). DNases occur in a number of species and any DNase capable of cleaving DNA may be used in the invention. The DNase may be from an animal source such as of bovine or porcine origin. It may be of plant, fungal, or microbial origin. However, typically and most preferably the DNase is of human origin and is preferably a recombinant human DNase. Commercially available DNase preparations such as Dornase^{™} and Pulmozyme^{™} may be used in embodiments of the invention.

The invention will be further illustrated by the following figures and examples. However, these examples and figures should not be interpreted in any way as limiting the scope of the present invention.

### FIGURES:

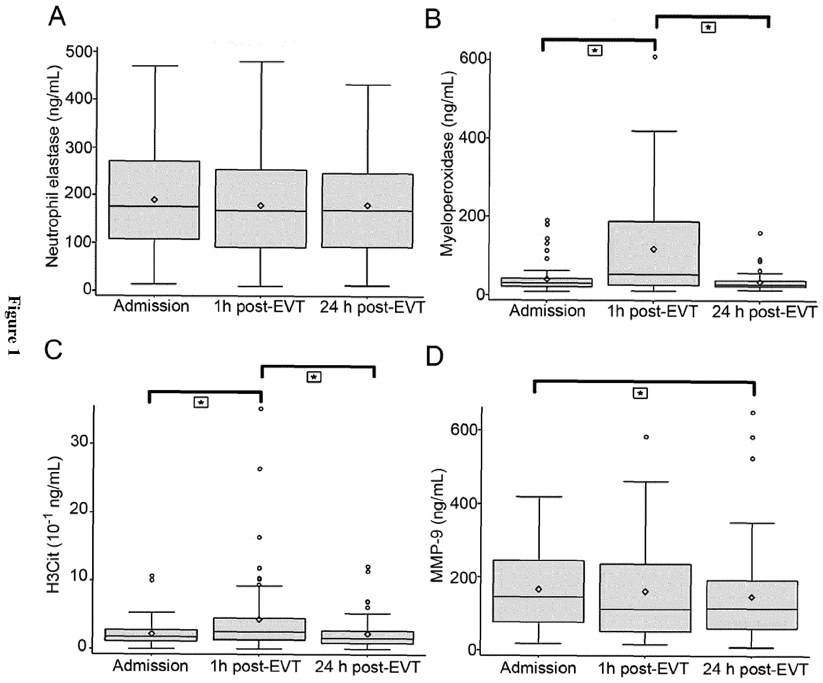

**Figure 1****: Distribution (Tukey's Box plots) of Neutrophil Parameters at admission, 1-hour, and 24-hour after endovascular treatment**
   * indicates P-value <0.05 from between time comparison in neutrophils parameters calculated using linear mixed model (an unstructured covariance pattern model) after Bonferroni correction. For MPO, MMP9 and H3c, a log-transformation was applied to satisfy the normality of model residuals. Diamonds indicates means values. Abbreviations: Abbreviations: EVT=endovascular treatment; H3c=citrullinated histone H3; MMP-9= matrix metalloproteinase-9 ; MPO= myeloperoxidase.

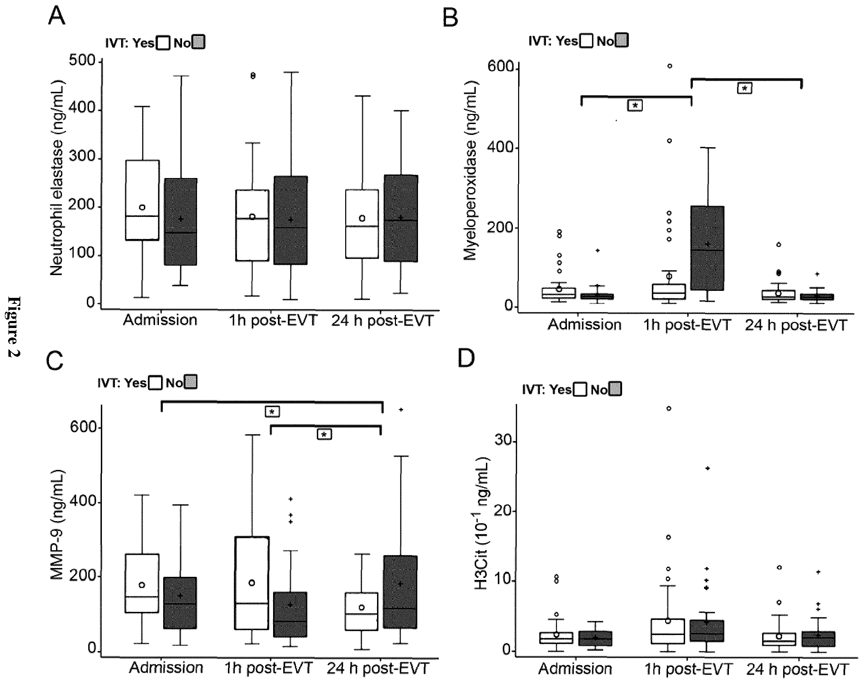

**Figure 2****. Neutrophil progression according to use or not of prior IVT**
   * indicates P-value<0.05 for post-hoc comparisons in variation of neutrophils between IV-treated and non-IV treated patients calculated using linear mixed model (an unstructured covariance pattern model) after Bonferroni correction. For MPO, MMP9 and H3CIT, a log-transformation was applied to satisfy the normality of model residuals. Abbreviations: EVT=endovascular treatment; H3c=citrullinated histone H3; IVT= intravenous thrombolysis; MMP-9= matrix metalloproteinase-9 ; MPO= myeloperoxidase;

### EXAMPLE:

### Methods

### Standard Protocol Approvals, Registrations, and Patient Consents

Patient information was collected prospectively using a standardized questionnaire (Endovascular Treatment in Ischemic Stroke (ETIS) registry). The local Ethics Committee approved this research protocol (CPP Ile de France VII, ID-RCB number: 2015-A01856-43).

### Patient data collection

All patients enrolled in this study were treated by EVT for a complete large vessel occlusion at Rothschild Foundation hospital between April 2016 and April 2018.

At admission, systolic, diastolic blood pressure and blood glucose were collected. Most patients preferentially underwent brain magnetic resonance imaging (MRI) using at least diffusion-weighted-imaging (DWI), T2 Fluid-Attenuation-Inversion-Recovery, 3D Time-of-Flight and T2 gradient-echo or susceptibility-weighted-imaging sequences. In cases of MRI contraindication, brain computed tomography (CT) scan with CT angiography were performed. The Alberta-Stroke-Program-Early-CT-Score (ASPECTS) was assessed for each patient by a neuroradiologist independent of the procedure using DWI sequence or CT scan.

Patients were treated in a dedicated neuroangiography suite with up-to-date equipment under conscious sedation or general anesthesia. The EVT procedure, a stent retriever and/or a direct aspiration first-pass technique, was chosen at the operator's discretion.

Time from symptom onset to recanalization was recorded. All patients had a Flat-Panel CT scan at the end of the procedure. Contrast enhancement in the Flat-Panel CT was interpreted as a blood-brain barrier (BBB) disruption.

Patients' clinical, radiological, biological and treatment characteristics were collected prospectively and are presented in supplemental Table 1.

CT scan or brain MRI were performed systematically 24 hours after EVT. Symptomatic intracranial hemorrhage (ICH) was assessed according to the ECASS III classification (European Cooperative Acute Stroke Study)¹¹ defined as any hemorrhage with neurological deterioration, as indicated by an NIHSS score ≥4 points higher than the baseline value, or any hemorrhage leading to death.

### Clinical Outcome

Clinical outcome was assessed with the modified Rankin Scale (mRS) at 90 days, during face-to-face interviews or via telephone conversations with the patient, their relatives, or their general practitioner. A poor clinical outcome was defined as a mRS score of 3 or more.

### Plasma collection

Blood was collected in citrate (BD Vacutainer 3ml, 0.129 M sodium citrate) and EDTA (BD Vacutainer 4ml, K3E 7.2mg) from arterial puncture at the beginning of the EVT procedure (before recanalization), at the end of EVT procedure (<1hour after recanalization) and from venous puncture 24 hours after EVT procedure. In patients that had received IVT prior to EVT, the first sample was collected after IVT. Blood cell counts including neutrophil count were systematically performed on the first blood sample. Plasma was collected after two centrifugations (10'x2000g at 20°C and 15'x2500g at 20°C) and immediately frozen at -80°C for further analysis.

### Quantification of neutrophil activation markers

Neutrophil activation markers were quantified using commercial ELISA kits for the following proteins: MPO (Hycult Biotech), matrix metalloproteinase-9 (MMP-9, R&D Systems), neutrophil elastase (Hycult Biotech), and citrullinated histone H3 (H3Cit, Cayman Chemical).

### Kinetics of histone citrullination and neutrophil extracellular trap formation

Neutrophils were isolated using gradient centrifugation, as described elsewhere¹⁹. Neutrophils were stimulated on a coverslip with 4 µM ionomycin and fixed with 3.7% PFA 30 minutes or 4 hours after treatment. Images were taken on a super-resolution optical microscope (Leica) with a 100x objective.

### Statistical analyses

Categorical variables were expressed as frequencies and percentages. Quantitative variables were expressed as mean ± standard deviation (SD), or median (interquartile range, IQR) for non-normal distribution. Normality of distributions was assessed graphically and by using the Shapiro-Wilk test. In first step, we assessed the intra-patients variability in neutrophil parameters (MPO, MMP-9, H3Cit and neutrophil elastase) from admission to 1- and 24-hours after endovascular treatment (EVT) using a linear mixed model (an unstructured covariance pattern model to take into account the correlation between repeated measures within the same subjects) with time as fixed categorical effect; post-hoc pairwise comparisons between each time interval were done using linear contrast. We further compared the intra-patients variability in neutrophil parameters between IV and non-IV treated patients prior EVT by using linear mixed model including time, IVT and time*IVT interaction as fixed effects; in case of significant interaction between time and IVT, post-hoc comparison in intra-patients variability between patients with and without IVT were done using linear contrast. Normality of model residuals were satisfied after a log-transformation for MPO, MMP3, and H3CIT. In a second step, we assessed the association of admission and variations between baseline and 1 hour or 24 hours after EVT on favorable outcome, hemorrhagic transformation and dramatic improvement by using logistic regression models, with a pre-specify adjustment on admission neutrophils count and admission NIHSS score for favorable outcome, and on IVT for hemorrhagic transformation. We checked the log-linearity assumption for all neutrophil parameters using restricted cubic spline function. Finally, we compared MPO admission and variations values according to favorable outcome and hemorrhagic transformations status in IVT-treated and non-IVT treated patients separately by using Mann-Whitney U test. We did not attempt to test heterogeneity across IVT subgroups regarding the small study sample. Statistical testing was done at the two-tailed α level of 0.05. Due to exploratory nature and the small sample size of the present study, we did not adjust for multiple comparisons, except for post-hoc pairwise comparisons in neutrophil parameters variations where a Bonferroni correction was applied. Data were analyzed using the SAS software package, release 9.4 (SAS Institute, Cary, NC).

### Data Availability

The datasets generated during and/or analyzed during the current study are not publicly available but are available from the corresponding author on reasonable request and with permission of all contributing authors.

### Results:

A total of 583 consecutive patients with an anterior circulation AIS were treated by EVT at our institution from April 2016 to April 2018. Among them, 72 randomly-selected patients had blood samples taken before, 1 hour after, and 24 hours after EVT, for analysis of circulating soluble markers of neutrophil activation. The median NIHSS score and DWI-ASPECTS at admission of the study group were of 16 (IQR, 10 to 21) and 8 (IQR, 5 to 8), respectively. Forty of the 72 studied patients (55.6%) had received IV tPA prior to EVT, and the median time from symptom onset to recanalization was of 309 minutes (IQR, 211 to 381). Successful recanalization (TICI 2b3) following EVT was achieved in 62 patients (86.1%). Overall, the demographics and clinical characteristics of the study group were similar to those of non-included EVT-treated patients admitted to the Rothschild Foundation hospital during the study period. Patients and treatment characteristics according IV-thrombolysis prior EVT use are resumed in **Table 1.**

### Dynamic changes in circulating neutrophil markers in EVT-treated patients

Plasma level of neutrophil elastase in AIS patients did not vary after EVT and remained stable up to 24 hour post-EVT (**Figure 1A****).** In contrast, plasma levels of both MPO and H3Cit raised significantly and transiently at 1 hour post-EVT, before returning to baseline admission values at 24 hour post-EVT (**Figure 1B and C**). A third type of evolution pattern was observed for MMP-9, whose plasma level fell below pre-EVT admission values at 24-hour post-EVT (Figure 1D).

The increase in circulating H3Cit as early as 1 hour post-EVT suggests that NET formation can expand rapidly in EVT-treated AIS patients. In agreement with this concept, we observed that citrullination of histone H3 occurred within 30 min after stimulating isolated neutrophils **(data not shown).**

### Admission citrullinated histone H3 level and dynamic changes in myeloperoxidase level are associated with 90-day functional outcome in EVT-treated patients

Among the circulating neutrophil activation markers analyzed, H3Cit was the only one whose admission level was associated with 90-day functional outcome, as determined by logistic regression analyses adjusted for admission neutrophil count and admission NIHSS score **(Table 2).** Lower admission H3Cit values were indeed associated with an increased likelihood of favorable outcome (adjusted odds ratio per 1 SD increase, 0.35, 95% CI, 0.13 to 0.93, p=0.036). Remarkably, dynamic changes in MPO level were also associated with 90-day functional outcome **(Table 2).** Patients with a poor functional outcome were characterized by a slight median increase in MPO level between admission and 24 hour post-EVT, whereas patients with a good outcome had a median decrease in MPO level in between admission and 24 hour post-EVT **(Table 2**). These differences in MPO level variations according to functional outcome were statistically significant (adjusted odds ratio per 1 SD increase, 0.37, 95% CI, 0.19 to 0.75, p=0.006, **Table 2**). A comparable but non-statistically significant association trend between MPO level variations and functional outcome was found for variations between admission and 1 hour post EVT (adjusted odds ratio per one SD increase, 0.79; 95%CI, 0.61 to 1.19, p=0.067, **Table 2).**

Similarly to good functional outcome, dramatic improvement was associated with lower H3Cit admission values (adjusted odds ratio per 1 SD increase, 0.34; 95% CI, 0.15 to 0.83, p=0.017, **Table 3**). The association between dramatic improvement and variations in MPO level from admission to 24 hour post-EVT approximated but did not reach statistical significance. The adjusted odds ratio for dramatic improvement per 1 SD increase in MPO level variations from admission to 24 hour post-EVT was of 0.63; 95% CI, 0.39 to 1.00, p=0.053 **(Table 3**).

There was no association between occurrence of hemorrhagic transformation and admission levels or changes in levels of any of the neutrophil activation markers tested (data not shown).

### Dynamic changes in circulating neutrophil markers in EVT-treated patients according to IVT status

We next compared dynamic changes in plasma levels of neutrophil markers according to IVT status. There was no difference in the evolution of neutrophil elastase level according to IVT status (**Figure 2A****).** The variations in MPO level from admission to 1 hour post-EVT, and from 1 hour to 24 hour post-EVT were significantly different between the IVT and non-IVT subgroups (**Figure 2B****).** As compared to the IVT subgroup, the non-IVT subgroup was characterized by a more important rise in MPO level at 1 hour post-EVT (**Figure 2B****).** Regarding MMP-9, the variations in its plasma level from admission to 24 hour post-EVT and from 1 hour to 24 hour post-EVT were significantly different according to IVT status (**Figure 2C**). Between admission and 1 hour post-EVT, median MMP-9 plasma level decreased in both subgroups (**Figure 2C**). However, they evolved in opposite directions in the following period of time, from 1 hour to 24 hour post-EVT. While the median MMP-9 plasma level continued decreasing until 24 hour post-EVT in the IVT subgroup, it raised during the same period in the non-IVT group (**Figure 2C**). There were no statistically significant differences in H3Cit plasma level evolution between the IVT and non-IVT subgroups (**Figure 2D**).

### Dynamic changes in myeloperoxidase levels following EVT are associated with functional outcome and hemorrhagic transformation in IVT-treated patients

Because IVT- and non-IVT-treated patients showed different patterns of MPO level evolution following EVT, with IVT strongly mitigating the plasma MPO peak at 1 hour post-EVT (**Figure 2B****),** we compared the clinical significance of MPO level evolution according to IVT status. There was no association between functional outcome or occurrence of hemorrhagic transformation and changes in MPO levels from admission to 1 hour or 24 hour post-EVT in non-IVT-treated patients **(Table 4**). Notably, the sharp increase in median MPO levels at 1 hour post-EVT in non-IVT-treated patients (**Figure 2D****)** occurred irrespectively of functional outcome or occurrence of hemorrhagic transformation **(Table 4**).

In IVT-treated patients, those with a good outcome or no hemorrhagic transformation displayed a median decrease in MPO plasma levels from admission to 1 hour and 24 hour post-EVT **(Table 4).** On the contrary, IVT-treated patients with a poor outcome or hemorrhagic transformation had a median elevation of MPO plasma levels during the same periods of time **(Table 4).** The differences in MPO plasma level variations from admission levels in IVT-treated patients according to functional outcome were statistically significant at both 1 hour and 24 hour post-EVT **(Table 4).** The differences in dynamic changes in MPO plasma levels between IVT-treated patients with and without hemorrhagic transformation were statistically significant for variations occurring between admission and 1 hour post-EVT, but not for those occurring between admission and 24 hour post-EVT **(Table 4).**

### Discussion:

In a context of increasing evidence that neutrophils represent a potential source of informative prognostic biomarkers in AIS patients, we investigated if and how circulating markers of neutrophil activation evolve in the first 24 h following EVT. Our results show that plasma levels of several neutrophil activation markers continue evolving after and despite successful removal of AIS thrombi by EVT. Among the most notable dynamic changes observed were an overall early and transient increase in MPO and H3Cit plasma levels at 1 h post-EVT, and a gradual decrease in MMP-9 plasma levels. In contrast, neutrophil elastase levels remained stable over time. Importantly, our results show that, as was previously described for admission values of MPO and H3Cit in non-EVT-treated AIS patients^{3,11}, dynamic changes in MPO levels in EVT-treated AIS patients also bear a prognostic value. Variations in MPO levels between admission and 24 hours post-EVT were indeed associated with 90-day functional outcome, independently of initial stroke severity and neutrophil count. During this time window, MPO levels rose more in patients with a poor functional outcome than in those with a good functional outcome. With respect to the prognostic value of admission levels of neutrophil activation markers, our results show that the association between higher admission levels of H3Cit and poor functional outcome that was previously described in non-EVT-treated AIS patients¹¹ also applies to EVT-treated patients. Admission plasma level of H3Cit thus appears as a biomarker that could help to identify patients that may need extra attention and support, irrespectively of the therapeutic strategy used for recanalization. From a pathophysiological perspective, the fact that high admission H3Cit is predictive of poor outcome irrespectively of the recanalization strategy suggests that neither EVT nor IVT is sufficient to counter the deleterious evolution associated with early NETosis in AIS patients. This situation may be seen as further evidence arguing in favor of the use of DNase as an adjuvant therapy to IVT and/or EVT at the acute phase of AIS. In contrast to the results of a previous study in non-EVT-treated patients³, we did not find any significant association between admission MPO values and functional outcome. This suggests that, as compared to admission H3Cit level, the predictive value of admission MPO level is less generic than that of H3Cit. It also indicates that the potential deleterious consequences associated with MPO secretion can be influenced by current patient management strategies. In support of this hypothesis, we observed that IVT prior to EVT resulted in a marked attenuation of the post-EVT MPO peak, suggesting that IVT may interfere with the detrimental thromboinflammatory cascade leading to MPO release in EVT-treated patients. Moreover, among patients that received IVT prior to EVT, those with a poor outcome or hemorrhagic transformation showed a lesser attenuation of their post-EVT MPO peak. Interestingly, in agreement with the present clinical data, we have shown previously that tPA infusion prior to recanalization help to reduce the thromboinflammatory response to LVO in a rat model of cerebral ischemia-reperfusion²⁰. The ability of IVT to limit MPO release in EVT-treated patients may account for the increased efficacy of bridging therapy over EVT alone^{21,22}.

Whereas IVT affected the evolution profile of MPO level in EVT-treated AIS patients, it did not impact that of H3Cit. There are several non-exclusive reasons that could explain the discrepancies in the evolution pattern and different susceptibility to IVT of the various neutrophil activation markers we analyzed here. First, they might reflect differences in the degree of cell-type specificity between these markers. For instance, as compared to MPO and neutrophil elastase, MMP-9 is less specific to neutrophils and can be expressed by a variety of cell types, including activated microglial cells in the cerebral infarct and peri-infarct areas of stroke patients²³. In addition, selective storage in and release from activated neutrophils, as well as differences in the clearance mechanisms between neutrophil activation markers²⁴⁻²⁶, may also account for their different evolution patterns.

Because the vast majority of the patients included in our study had successful recanalization (>86 % of patients reaching a TICI score of 2b3), it remains unknown whether the post-EVT MPO and H3Cit peaks are consequences of the recanalization process or parts of the natural course of the disease. Nevertheless, the fact that baseline levels of neutrophil activation markers like MPO and H3Cit were previously shown to be increased in AIS patients^{3,11} indicates that initiation of neutrophil activation is not a consequence of recanalization EVT. One cannot exclude, however, that recanalization amplifies neutrophil activation and NETosis. Furthermore, as we confirm here, NETosis is a rapid phenomenon¹⁹, with histone citrullination occurring within 30 min following neutrophil stimulation, a kinetics compatible with an increase in H3Cit levels rapidly after stroke onset. That neutrophil activation has already begun at patient admission and can continue evolving rapidly after EVT has potential therapeutic implications. In fact, it suggests that strategies aiming at preventing neutrophil activation have little chance of efficacy unless initiated early during the course of AIS patient management. Treatments targeting neutrophil effectors like NETs or MPO rather than neutrophil activation pathways may offer longer therapeutic windows.

In conclusion, our results show that admission H3Cit level and changes in MPO level are associated with outcome in EVT-treated AIS patients, and support the notion that neutrophil effectors are informative biomarkers of AIS severity and prognosis, as well as possible mediators of cerebral injury in AIS patients. Further investigations are needed to determine the actual impact of EVT-mediated recanalization on neutrophil activation in those patients.

### TABLES:

**Table 1. Patients and Treatment Characteristics according Iv-thrombolysis prior EVT use.**

| IV-thrombolysis prior EVT | | | |
|---|---|---|---|
| **Characteristics** | No (n=32) | Yes (n=40) | ASD |
| **Baseline demographics and medical history** | | | |
| Age, y, mean ± SD | 73.0 ± 15.3 | 69.9 ± 18.0 | 18.9 |
| Men | 17/32 (53.1) | 19/40 (47.5) | 11.3 |
| Hypertension | 21/32 (65.6) | 22/39 (56.4) | 19.0 |
| Diabetes | 9/32 (28.1) | 6/39 (15.4) | 31.2 |
| Current smoking | 6/29 (20.7) | 3/39 (7.7) | 37.9 |
| Antithrombotic medications | | | |
| *Antiplatelet* | 4/31 (12.9) | 8/39 (20.5) | 20.5 |
| *Anticoagulant* | 12/31 (38.7) | 2/39 (5.1) | 88.8 |

| **Current stroke event** | | | |
|---|---|---|---|
| Pre-stroke mRS ≥1 | 7/32 (21.9) | 6/39 (15.4) | 16.7 |
| Site of occlusion | | | |
| *M1-MCA* | 17/32 (53.1) | 22/40 (55.0) | NA |
| *M2-MCA* | 2/32 (6.3) | 6/40 (15.0) | |
| *T-car* | 7/32 (21.9) | 6/40 (15.0) | |
| *Tandem* | 1/32 (3.1) | 4/40 (10.0) | |
| *Vertebro-basiliar* | 2/32 (6.3) | 1/40 (2.5) | |
| *Extracranial ICA* | 2/32 (6.3) | 1/40 (2.5) | |
| *Others* | 1/32 (3.1) | 0/40 (0.0) | |
| Admission NIHSS score, median (IQR) | 16 (10 to 21) | 17 (9 to 21) | 1.1 |
| ASPECTS score, median (IQR)^{a} | 8 (5 to 9) | 8 (5 to 8) | 19.7 |

| **Treatment characteristics** | | | |
|---|---|---|---|
| Number of devices passes, median (IQR)^{b} | 2 (1 to 4) | 3 (1 to 4) | 30.6 |
| Procedural times, min, median (IQR) | | | |
| *Onset to puncture* | 251 (163 to 328) | 229 (165 to 304) | 16.7 |
| *Imaging to groin puncture^{a}* | 82 (51 to 150) | 108 (65 to 161) | 24.9 |
| *Onset to recanalization^{a}* | 315 (224.0 to 427.0) | 299 (207 to 359) | 28.9 |

| | | | |
|---|---|---|---|
| Values are expressed as number (%) unless otherwise indicated. ^{a}1 missing value, ^{b}2 missing values. Abbreviations: ASPECTS= Alberta stroke program early computed tomography score, EVT= endovascular treatment, ICA=intracranial carotid artery, IQR=interquartile range, MCA=middle cerebral artery, mRS= modified Ranking scale, NIHSS=national institutes of health stroke scale, SD=standard deviation, ASD= absolute standardized difference. | | | |

**Table 2. Associations of neutrophils parameters (admission and variation between baseline and 1 hour or 24 hours after EVT) with 90-day functional outcome.**

| **Neutrophils Parameters** | **90-day Functional Outcome** | | | |
|---|---|---|---|---|
| | Poor outcome (n=42) | Good outcome(n=30) | OR (95%CI)*† | P-Valuet† |
| MPO | | | | |
| Admission | 33.2 (21.4 to 49.9) | 28.1 (22.6 to 32.8) | 1.23 (0.63 to 2.39) | 0.55 |
| Variation between baseline and 1 hour after EVT | 33.1 (-1.4 to 180.7) | 0.9 (-6.2 to 93.3) | 0.79 (0.61 to 1.19) | 0.067 |
| Variation between baseline and 24 hour after EVT | 0.9 (-5.4 to 11.0) | -1.6 (-7.1 to 0.2) | 0.37 (0.19 to 0.75) | 0.006 |

| MMP9 | | | | |
|---|---|---|---|---|
| Admission | 166 (109 to 260) | 116 (62.2 to 166) | 0.78 (0.40 to 1.53) | 0.47 |
| Variation between baseline and 1 hour after EVT | -39.6 (-94.3 to 119) | -31.5 (-71.9 to 32.4) | 0.43 (0.17 to 1.08) | 0.071 |
| Variation between baseline and 24 hour after EVT | -51.8 (-144 to 40.0) | -43.7 (-88.6 to -17.0) | 0.59 (0.20 to 1.75) | 0.34 |

| H3CIT | | | | |
|---|---|---|---|---|
| Admission | 2.1 (1.5 to 3.0) | 1.2 (0.6 to 2.1) | 0.35 (0.13 to 0.93) | 0.036 |
| Variation between baseline and 1 hour after EVT | 0.9 (-0.3 to 2.2) | 0.7 (0.0 to 1.7) | 0.99 (0.99 to 1.01) | 0.76 |
| Variation between baseline and 24 hour after EVT | -0.1 (-1.0 to 0.6) | 0.2 (-0.4 to 0.7) | 1.00 (0.99 to 1.01) | 0.97 |
| Elastase | | | | |
| Admission | 177 (107 to 287) | 157 (110 to 267) | 1.29 (0.68 to 2.43) | 0.44 |
| Variation between baseline and 1 hour after EVT | -16.6 (-113 to 70.6) | -14.8 (-24.3 to 34.4) | 0.84 (0.51 to 1.40) | 0.51 |
| Variation between baseline and 24 hour after EVT | 8.9 (-40.8 to 79.1) | -14.8 (-67.4 to 31.6) | 0.73 (0.44 to 1.22) | 0.24 |

| | | | | |
|---|---|---|---|---|
| Values (ng/mL) are expressed as median (IQR). Good outcome is defined as 90-day mRS 0-2 or equal to pre-stroke. *OR expressed per one SD increase in neutrophil parameters. †calculated using logistic regression models adjusted for initial NIHSS score and neutrophil count. Abbreviations: EVT=endovascular treatment; H3c=citrullinated histone H3; IVT= intravenous thrombolysis; MMP-9= matrix metalloproteinase-9 ; MPO= myeloperoxidase; mRS=modified ranking scale; OR= odds ratio. | | | | |

**Table 3. Associations of neutrophils parameters (admission and variation between baseline and 1 hour or 24 hours after EVT) with dramatic improvement**

| **Parameters** | **Dramatic improvement** | | | |
|---|---|---|---|---|
| | No (n=46) | Yes (n=26) | OR (95%CI) *† | p-valuest† |
| MPO | | | | |
| Admission | 30.9 (23.3 to 41.7) | 28.4 (18.8 to 43.7) | 0.83 (0.48 to 1.45) | 0.52 |
| Variation between baseline and 1 hour after EVT | 34.7 (-1.2 to 173) | 0.0 (-6.6 to 81.7) | 0.96 (0.84 to 1.10) | 0.60 |
| Variation between baseline and 24 hour after EVT | 0.7 (-4.1 to 9.4) | -3.5 (-12.0 to 0.1) | 0.63 (0.39 to 1.00) | 0.053 |

| MMP9 | | | | |
|---|---|---|---|---|
| Admission | 148(101 to 260) | 132 (40 to 198) | 0.58 (0.32 to 1.04) | 0.067 |
| Variation between baseline and 1 hour after EVT | -46.3 (-88.6 to 41.8) | 2.8 (-94.3 to 42.9) | 0.98 (0.49 to 1.69) | 0.95 |
| Variation between and 24 hour after EVT baseline | -36.0 (-88.6 to 80.4) | -71.9 (-149 to -15.0) | 0.61 (0.22 to 1.72) | 0.35 |

| H3CIT | | | | |
|---|---|---|---|---|
| Admission | 2.1 (1.5 to 3.1) | 1.3 (0.7 to 1.8) | 0.34 (0.15 to 0.83) | 0.017 |
| Variation between and 1 hour after EVT baseline | 0.6 (-0.6 to 2.0) | 1.2 (0.3 to 1.8) | 1.00 (0.99 to 1.01) | 0.20 |
| Variation between and 24 hour after EVT baseline | -0.1 (-1.3 to 0.6) | 0.3 (-0.3 to 0.9) | 1.01 (0.99 to 1.02) | 0.064 |

| Elastase | | | | |
|---|---|---|---|---|
| Admission | 183 (112 to 274) | 155 (81.3 to 267) | 0.75 (0.44 to 1.27) | 0.28 |
| Variation between and 1 hour after EVT baseline | -15.4 (-88.7 to 44.7) | -12.4 (-39.2 to 55.5) | 1.19 (0.81 to 1.74) | 0.37 |
| Variation between and 24 hour after EVT baseline | 1.6 (-36.9 to 37.7) | -19.0 (-98.0 to 79.1) | 1.07 (0.68 to 1.68) | 0.77 |

| | | | | |
|---|---|---|---|---|
| Values (ng/mL) are expressed as median (IQR). *OR expressed per one SD increase in neutrophil parameters. †calculated using logistic regression. Abbreviations: EVT=endovascular treatment; H3c=citrullinated histone H3; IVT= intravenous thrombolysis; MMP-9= matrix metalloproteinase-9 ; MPO= myeloperoxidase; mRS=modified ranking scale; OR= odds ratio. | | | | |

**Table 4. Comparison of MPO level at admission and of dynamic changes in MPO level within 1 hour and 24 hours post-EVT according to 90-day favorable outcome and 24-hour hemorrhagic transformation in IVT-treated and non-IVT treated patients**

| MPO | IVT | n | Poor outcome | n | Good outcome | p-values |
|---|---|---|---|---|---|---|
| Admission | No | 19 | 30.6 (21.5 to 37.8) | 11 | 24.3 (21.0 to 28.6) | 0.23 |
| | Yes | 21 | 36.8 (21.3 to 51.0) | 18 | 29.7 (23.9 to 41.3) | 0.36 |
| Variation between baseline and 1 hour after EVT | No | 19 | 135.6 (17.5 to 254.7) | 10 | 124.9 (65.9 to 197.7) | 0.87 |
| | Yes | 19 | 11.6 (-3.2 to 72.2) | 17 | -1.9 (-8.8 to 0.9) | 0.029 |
| Variation baseline and after EVT between 24 hour | No | 18 | 0.6 (-7.2 to 4.9) | 11 | -1.2 (-3.0 to 4.8) | 0.95 |
| | Yes | 21 | 2.7 (-4.7 to 12.6) | 17 | -3.8 (-18.8 to 0.1) | 0.016 |

| MPO | IVT | n | No hemorrhagic transformation | n | Hemorrhagic transformation | p-values |
|---|---|---|---|---|---|---|
| Admission | No | 21 | 24.3 (20.7 to 32.4) | 9 | 30.5 (28.2 to 34.0) | 0.21 |
| | Yes | 30 | 30.9 (23.1 to 42.0) | 8 | 46.3 (33.7 to 76.8) | 0.082 |
| Variation baseline and after EVT between 1 hour | No | 20 | 106.2 (10.2 to 213.1) | 9 | 172.8 (96.9 to 302.5) | 0.25 |
| | Yes | 27 | -1.9 (-8.8 to 1.6) | 8 | 29.1 (-0.3 to 55.1) | 0.036 |
| Variation baseline and after EVT between 24 hour | No | 20 | 0.6 (-4.8 to 6.7) | 9 | -1.4 (-4.0 to 0.7) | 0.46 |
| | Yes | 29 | -1.7 (-18.8 to 1.0) | 8 | 3.4 (-2.3 to 23.0) | 0.16 |

| | | | | | | |
|---|---|---|---|---|---|---|
| Values (ng/mL) are expressed as median (IQR). Good outcome is defined as 90-day mRS 0-2 or equal to pre-stroke. P-values were calculated using Mann-Whitney U test. Abbreviations: EVT=endovascular treatment; H3c=citrullinated histone H3; IVT= intravenous thrombolysis; MMP-9= matrix metalloproteinase-9 ; MPO= myeloperoxidase; mRS=modified ranking scale; OR= odds ratio. | | | | | | |

### REFERENCES:

Throughout this application, various references describe the state of the art to which this invention pertains. The disclosures of these references are hereby incorporated by reference into the present disclosure.
1. Badhiwala JH, Nassiri F, Alhazzani W, et al. Endovascular thrombectomy for acute ischemic stroke ameta-analysis. JAMA - J. Am. Med. Assoc. 2015;314(17): 1832-1843.
2. Boisseau W, Desilles JP, Fahed R, et al. Neutrophil count predicts poor outcome despite recanalization after endovascular therapy. Neurology 2019;93(5):e467-e475.
3. Maestrini I, Tagzirt M, Gautier S, et al. MPO is partially associated with neutrophil deleterious effect in acute cerebral ischemia. Neurology 2020;10.1212/WNL.0000000000009179.
4. Maestrini I, Strbian D, Gautier S, et al. Higher neutrophil counts before thrombolysis for cerebral ischemia predict worse outcomes. Neurology 2015;85(16):1408-1416.
5. Jickling GC, Dziedzic T. Neutrophil count is related to stroke outcome following endovascular therapy. Neurology 2019;93(5): 194-195.
6. Brooks SD, Spears C, Cummings C, et al. Admission neutrophil-lymphocyte ratio predicts 90 day outcome after endovascular stroke therapy. J. Neurointerv. Surg. 2014;6(8):578-583.
7. Pikija S, Sztriha LK, Killer-Oberpfalzer M, et al. Neutrophil to lymphocyte ratio predicts intracranial hemorrhage after endovascular thrombectomy in acute ischemic stroke. J. Neuroinflammation 2018;15(1)
8. Lux D, Alakbarzade V, Bridge L, et al. The association of neutrophil-lymphocyte ratio and lymphocyte-monocyte ratio with 3-month clinical outcome after mechanical thrombectomy following stroke. J. Neuroinflammation 2020;17(1): 1-9.
9. Desilles JP, Meseguer E, Labreuche J, et al. Diabetes mellitus, admission glucose, and outcomes after stroke thrombolysis: A registry and systematic review. Stroke 2013;44(7):1915-1923.
10. Desilles JP, Syvannarath V, Ollivier V, et al. Exacerbation of Thromboinflammation by Hyperglycemia Precipitates Cerebral Infarct Growth and Hemorrhagic Transformation. Stroke 2017;48(7): 1932-1940.
11. Vallés J, Lago A, Santos MT, et al. Neutrophil extracellular traps are increased in patients with acute ischemic stroke: Prognostic significance. Thromb. Haemost. 2017;117(10): 1919-1929.
12. Tay A, Tamam Y, Yokus B, et al. Serum myeloperoxidase levels in predicting the severity of stroke and mortality in acute ischemic stroke patients. Eur. Rev. Med. Pharmacol. Sci. 2015;19(11):1983-1988.
13. Laridan E, Denorme F, Desender L, et al. Neutrophil extracellular traps in ischemic stroke thrombi. Ann. Neurol. 2017;82(2):223-232.
14. Ducroux C, Di Meglio L, Loyau S, et al. Thrombus neutrophil extracellular traps content impair tPA-induced thrombolysis in acute ischemic stroke. Stroke 2018;49(3):754-757.
15. Di Meglio L, Desilles JP, Ollivier V, et al. Acute ischemic stroke thrombi have an outer shell that impairs fibrinolysis. Neurology 2019;93(18):E1686-E1698.
16. Denorme F, Manne BK, Portier I, et al. Platelet necrosis mediates ischemic stroke outcome in mice. Blood 2020;135(6):429-440.
17. del Zoppo GJ. Microvascular changes during cerebral ischemia and reperfusion. Cerebrovasc. Brain Metab. Rev. 1994;6(1):47-96.
18. Desilles JP, Syvannarath V, Meglio L Di, et al. Downstream microvascular thrombosis in cortical venules is an early response to Proximal Cerebral Arterial occlusion. J. Am. Heart Assoc. 2018;7(5)
19. Thiama HR, Wong SL, Qiu R, et al. NETosis proceeds by cytoskeleton and endomembrane disassembly and PAD4-mediated chromatin decondensation and nuclear envelope rupture. Proc. Natl. Acad. Sci. U. S. A. 2020;117(13):7326-7337.
20. Desilles JP, Loyau S, Syvannarath V, et al. Alteplase Reduces Downstream Microvascular Thrombosis and Improves the Benefit of Large Artery Recanalization in Stroke. Stroke 2015;46(11):3241-3248.
21. Gariel F, Lapergue B, Bourcier R, et al. Mechanical thrombectomy outcomes with or without intravenous thrombolysis insight from the ASTER randomized trial. Stroke 2018;49(10):2383-2390.
22. Maria F Di, Mazighi M, Kyheng M, et al. Intravenous thrombolysis prior to mechanical thrombectomy in acute ischemic stroke: Silver bullet or useless bystander? J. Stroke 2018;20(3):385-393.
23. Rosell A, Ortega-Aznar A, Alvarez-Sabin J, et al. Increased brain expression of matrix metalloproteinase-9 after ischemic and hemorrhagic human stroke. Stroke 2006;37(6):1399-1406.
24. Borregaard N, Cowland JB. Granules of the human neutrophilic polymorphonuclear leukocyte. Blood 1997;89(10):3503-3521.
25. Papayannopoulos V, Metzler KD, Hakkim A, Zychlinsky A. Neutrophil elastase and myeloperoxidase regulate the formation of neutrophil extracellular traps. J. Cell Biol. 2010;191(3):677-691.
26. Witko-Sarsat V, Rieu P, Descamps-Latscha B, et al. Neutrophils: Molecules, functions and pathophysiological aspects. Lab. Investig. 2000;80(5):617-654.
27. Desilles JP, Mazighi M, Ho-Tin-Noé B. Letter by desilles et al regarding article, "ischemia-Reperfusion Injury after Endovascular Thrombectomy for Ischemic Stroke." Stroke 2019;50(3):E98.
28. Gauberti M, Lapergue B, De Lizarrondo SM, et al. Ischemia-reperfusion injury after endovascular thrombectomy for ischemic stroke. Stroke 2018;49(12):3071-3074.
29. Stegner D, Klaus V, Nieswandt B. Platelets as Modulators of Cerebral Ischemia/Reperfusion Injury. Front. Immunol. 2019; 10

## Claims

1. A method of predicting the outcome of a patient suffering from an acute ischemic stroke (AIS) comprising determining the level of citrullinated histones H3 in a sample obtained from the patient wherein said level indicates the outcome.

2. The method of claim 1 for predicting the outcome of a patient suffering from an acute ischemic stroke (AIS) after endovascular therapy (EVT).

3. The method of claim 1 for predicting the functional outcome of the patient.

4. The method of claim 1 wherein the sample is obtained at admission of the patient.

5. The method of claim 1 wherein the sample is a blood sample.

6. The method of claim 1 wherein level of citrullinated histones H3 in the sample is determined by a quantitative immunoassay.

7. The method of claim 1 wherein high levels of H3Cit indicate a poor outcome (e.g. poor functional outcome) and conversely low levels of H3Cit indicate a good outcome (e.g. good functional outcome).

8. The method of claim 1 that comprises the steps of i) quantifying the level of H3Cit in the sample obtained from the patient ii) comparing the level quantified at step i) with a predetermined reference value and iii) concluding that the patient will have a poor outcome when the level quantified at step i) is higher than the predetermined reference value or inversely concluding that the patient will have a good outcome when the content quantified at step i) is lower than the predetermined reference value.

9. The method of claim 1 wherein the patient is eligible with a therapy with DNAse when it is concluded that the patient will a poor outcome.
